# EUROPEAN PATENT SPECIFICATION

(11) **EP 0 167 366 B1**
(45) Date of publication and mention of the grant of the patent: **23.04.1997**
(21) Application number: 85304635.7
(22) Date of filing: 28.06.1985
(51) Int. Cl.: C07H 21/00

(54) **Accelerated nucleic acid reassociation method**
Schnellere Methode für die Reassoziation von Nukleinsäure
Méthode accélérée pour la réassociation d'acides nucléiques

(30) Priority: 05.07.1984 US 627795
(43) Date of publication of application: 08.01.1986
(73) Proprietor: GEN-PROBE INCORPORATED, San Diego California 92121-1514 (US)
(72) Inventor: Kohne, David Edward, La Jolla California 92037 (US)
(74) Representative: Goldin, Douglas Michael

(56) References cited:
- EP-A- 0 127 327
- WO-A-84/02721
- CHEMICAL ABSTRACTS, vol. 87, no. 25, December 1977, page 331, abstract no. 196819n, Columbus, Ohio, US; D.E. KOHNE et al.: "Room temperature method for increasing the rate of DNA reassociation by many thousandfold: the phenol emulsion reassociation technique", & BIOCHEMISTRY 1977, 16(24), 5329-41

## Description

The present invention is directed to a method for the renaturation, reassociation or hybridization of single stranded nucleic acid molecules into double stranded nucleic acid molecules wherein the rate of reaction is greatly increased over the rate of reaction under standard reference conditions of 0.12M phosphate buffer at 60°C. More particularly, the present invention is directed to a method for the renaturation, reassociation or hybridization of nucleic acids, including DNA to DNA, RNA to DNA, and RNA to RNA reactions wherein the rate of the reaction is greatly increased by factors of 100 times, and even up to several thousand times that of the reaction rates observed under standard reference conditions. These greatly accelerated reaction rates are achieved through the utilization of reaction solutions containing nucleic acid precipitating agents.

### Description of the Prior Art

Numerous methods for the nucleation of single stranded nucleic acid molecules into double stranded molecules are known in the art and have proven to be useful tools for the analysis of genetic material from a wide variety of organisms. Generally speaking, these nucleation reactions, renaturation, reassociation and hybridization, are based on the tendency of single stranded nucleic acid molecules having blocks or segments of complementary base sequences to form base paired structures between these complementary sequences and to rewind forming double helices. The greater the extent of sequence complementarity between the single stranded nucleic acid molecules, the greater the tendency for a given pair of molecules to nucleate and form a double stranded or duplex molecule.

Renaturation, reassociation and hybridization are essentially synonymous reactions between single stranded nucleic acid molecules. As such, they will be discussed interchangeably throughout the body of this paper. However, the following distinction may prove helpful in understanding the technology involved. Renaturation generally refers to the formation of a double stranded nucleic acid molecule from two single stranded molecules which were originally base paired to one another and separated thorugh a denaturation process. Reassociation refers to the process of forming double stranded nucleic acid molecules between two single stranded molecules which usually come from different original molecules. Hybridization refers to the formation of double stranded nucleic acid molecules from single stranded nucleic acid molecules of individual origin with respect to one another. It should be appreciated that these are not clear cut distinctions and that there is considerable overlap between them. For example, DNA:DNA reactions are commonly called both reassociation and hybridization reactions. On the other hand, the formation of an RNA:RNA double stranded molecule is generally referred to as hybridization.

The kinetics of these reactions are well understood in the art as following second-order kinetics. Thus, as the concentration of the single stranded nucleic acid molecules is increased, the rate of the reaction is also increased. Conversely, decreasing the concentration of the single stranded nucleic acid reactant will decrease the rate of reaction and thus increase the time necessary for the formation of the double stranded nucleic acid molecules to take place.

The effect of temperature on the reaction rate is also well known in the art. As the temperature of the reaction decreases below the Tₘ (the temperature at which 50% of the double stranded molecules is denatured, also known as the "melting temperature") a maximum rate for the reaction is achieved at temperatures of approximately 15°C to 30°C below the Tₘ. Further decreases in temperature are known to decrease the rate below this maximum rate.

Lastly, with respect to the kinetics of these reactions, it is known that the reaction rates are very dependent on the ionic strength below 0.4M for electrolytes such as NaCl and are almost independent of the salt concentration above this ionic strength.

More information on the kinetics and reaction rates of these nucleic acid association reactions can be found in the following publications:
Wetmur, R., and Davidson, N. (1968), J. Molc. Biol. 31, 349;
Wetmur, R., (1975), Biopolymers 14, 2517;
Britten, R., J., Graham, D., and Neufeld, B. (1974), Methods Enzymol. 29, 363;
Kohne, D. E., Levison, S. A., and Byers, M. J. (1977) Biochemistry 16, 5329; and
Orosz, J. M., and Wetmur, J. G., (1977) Biopolymer 16, 1183.

It has long been recognized in the art that a major limitation on the utility of these known nucleic acid association techniques is the basic rate of the reaction. Reaction times on the order of several hours to tens of hours and even days are commonplace. Increasing the reaction rate by increasing the quantities of single stranded nucleic acid molecules utilized in the reactions (due to the second-order kinetics) is not a desirable solution to this problem for three reasons. First, in many cases the target single stranded nucleic acid in the reaction is extracted from physiological samples which inherently limits the amount of such nucleic acid available to that contained in the cells of the physiological sample. Secondly, there are significant expenses associated with the use of nucleic acid reactants which limits the practical utility of increasing the quantity of reactants. Thirdly, increasing the quantities of single stranded nucleic acid molecules decreases the sensitivity of the reaction by increasing the background noise. Nonetheless, a number of techniques have been developed to increase the basic rate of these reactions by factors of 5 to 50 or more. Techniques of limited applicability have also been developed which increase the basic reaction rate by factors on the order of 1000 or more. However, as will be discussed in detail below, none of these prior art techniques has been successful at producing greatly accelerated reaction rates of 100 times or more than the basic reference standard reaction rate in a single phase system applicable to DNA:DNA, DNA:RNA, and RNA:RNA reactions.

When dealing with reaction rates, the accepted standard reference condition for the comparison of these rates is an aqueous solution of 0.12M phosphate buffer (PB) at 60°C. A similar standard reference condition that is often used giving comparable reaction rates is an aqueous solution of 0.18M NaCl at 60°C.

By far the most common technique of accelerating the reaction rate above that of the standard reference condition has been to increase the salt concentration of the reaction solution above that of the standard reference condition. As detailed in the following table, numerous researchers have been successful in promoting rate increases on the order of 10 fold or more above the standard reference condition reaction rate by increasing the salt concentrations of their reaction solutions well above the standard reference condition concentration. However, as demonstrated by the following table, while significant reaction rate increases are observed by increasing the salt concentration, the prior art techniques indicate that the rate of increase levels off or even falls for salt concentrations above 2M.

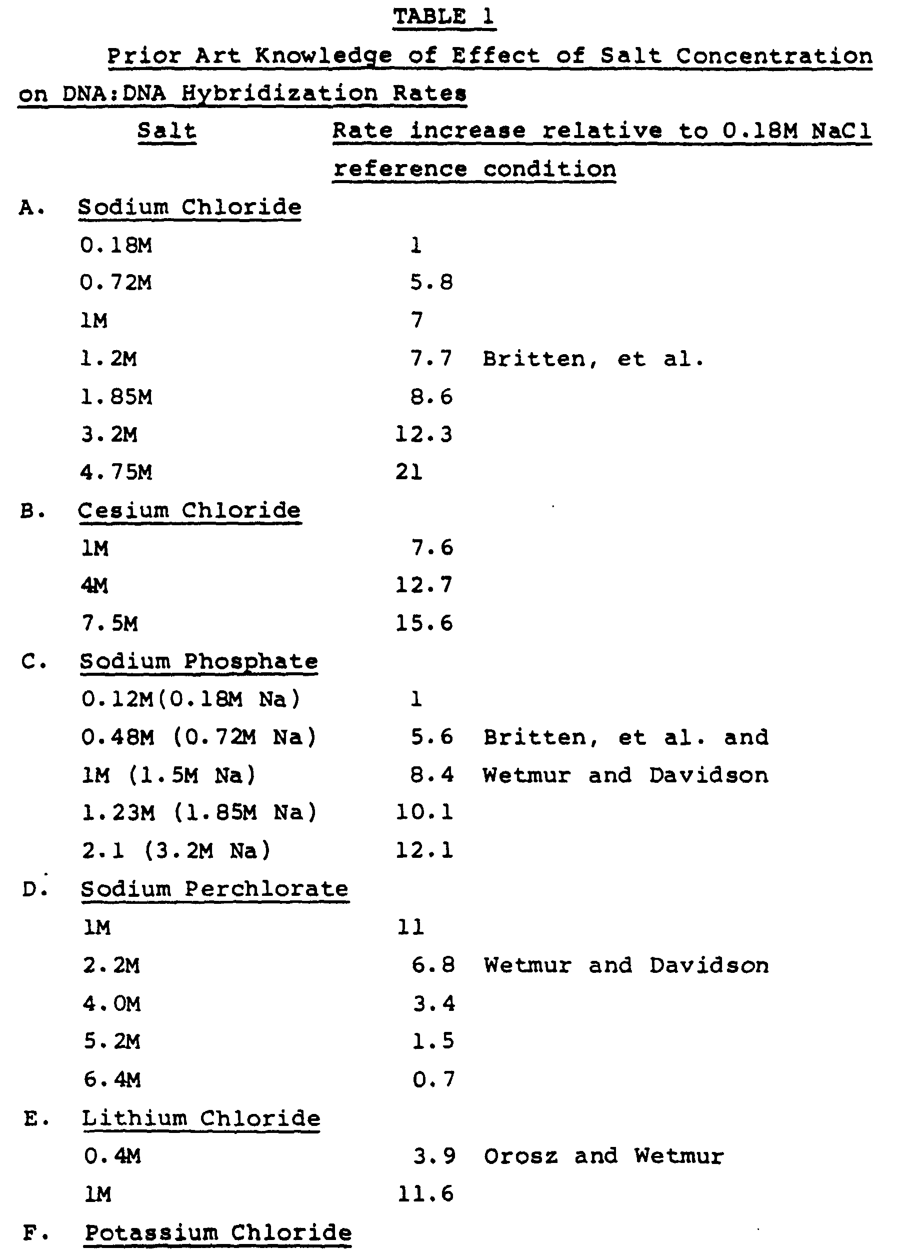

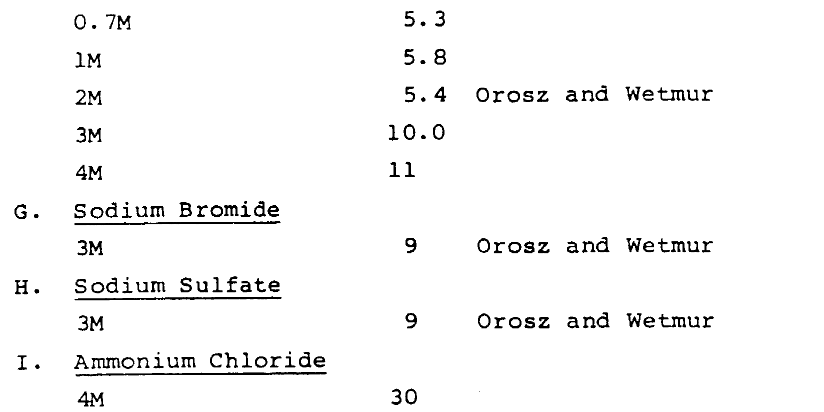

While the data in Table 1 relates to the rate increases found with respect to DNA:DNA reactions, it will be appreciated that the reaction rates of RNA with DNA are reported as being less affected by changes in salt contentration. Other researchers have demonstrated that for salt concentrations above the standard reference conditions, the relative rate of RNA:DNA reaction is affected to about one-half the extent of those rates found for DNA:DNA reactions when the RNA used has comparatively little secondary structure. For RNA reactants with more secondary structure, the effect of elevated salt concentration has been found to be even less. In fact, no change in rate is observed for hybridization of excess RNA with DNA over comparative ranges of salt concentrations (see, e.g., Van Ness, J. and Hahn, W. E. (1982) Nucl. Acids. Res. 10, 8061). While little data is available for RNA:DNA hybridization where the DNA is the excess reactant, it is commonly assumed in the art that the effect of elevated salt concentration on such a reaction system is comparable to that of the excess RNA system.

An alternative approach to the acceleration of the rate of these nucleic acid association reactions is the previously developed two-phase phenol aqueous emulsion technique for the reassociation of DNA to DNA (Kohne, D. E., Levinson, S. A., and Byers, M. J. (1977) Biochemistry 16, 5329). In this two-phase system, the agitation of an emulsion formed between phenol and an aqueous salt solution has produced greatly accelerated reaction rates over 100 times faster than comparative standard condition rates. However, the two-phase phenol emulsion technique has not produced similarly greatly accelerated reaction rates for RNA:RNA and RNA:DNA systems. The greatest reaction rate increase observed for RNA:RNA and RNA:DNA reactions is only 50 to 100 times that of the standard reference condition rate. This technique is further limited in that reaction will not occur unless an emulsion is present and agitated and the reaction temperature is below 75°C.

A number of other techniques for producing reaction rate increases on the order of 10 fold above the standard reference condition rate have utilized the volume exclusion principle to promote the acceleration of the reaction rate. These techniques utilize the synthetic polymers polyethylene glycol, dextran, or dextran sulfate to reduce the volume of reaction solution available to the nucleic acid reactants and thereby increase their effective concentration. However, while reaction rate increases of 10 to 15 fold over the standard reference condition rate for DNA:DNA reactions have been reported, rate increases of only about 3 fold are reported for RNA:DNA reactions. Details of these techniques can be found in the following publications:
Renz, M., and Kurz, C. (1984) Nucl. Acids Res. 12, 3435; and
Wahl, G. M., Stern, M., and Stark, G. R. (1979) Proc. Natl. Acad. Sci. USA 76, 3683.

Our International Patent Application W084/02721 published on 19th July 1984 (prior art under Art. 54(3)EPC) describes a method for detecting and quantitating organisms by contacting their nucleic acid with a marked probe comprising nucleic acid molecules complementary to the RNA or other nucleic acid sequences of the organism under nucleic acid hybridisation conditions and determining the degree of hybridisation that has occurred.

Accordingly, it is a principal object of the present invention to provide a method for the renaturation, reassociation, or hybridization of nucleic acids that produces a greatly accelerated reaction rate on the order of 100 or more times that of the standard reference condition rate and that is applicable to DNA:DNA, RNA:DNA, and RNA:RNA reaction systems. Additionally, it is a further object of the present invention to provide a method that promotes greatly accelerated reaction rates without requiring the utilization of a two-phase system or the formation of an emulsion. It is a further object of the present invention to provide a method wherein greatly accelerated reaction rates are obtainable without the need to increase the concentrations of single stranded nucleic acid reactants. Lastly, it is an additional object of the present invention to provide a method for greatly accelerating the rate of these nucleic acid association reactions that is widely applicable to a variety of reaction mixture volumes and hybridization temperatures.

### SUMMARY OF THE INVENTION

Generally stated, the present invention accomplishes the above described objectives by providing a method for the formation of double stranded nucleic acid molecules from separate single stranded nucleic acid molecules wherein a single phase reaction solution incorporating a known concentration of at least one nucleic acid precipitating agent is utilized to greatly increase the reaction rate over the standard reference condition reaction rate. The improved method of the present invention is widely applicable to a broad range of reaction solution volumes and nucleic acid concentrations and promotes reaction rates on the order of 100 to 1000 fold greater than the standard reference condition reaction rate for DNA:DNA, RNA:DNA and RNA:RNA reactions.

More particularly, the method of the present invention comprises the steps of preparing an aqueous reaction solution containing complementary single stranded nucleic acids, one of which preferably incorporates a detectable marker, and a known concentration of at least one nucleic acid precipitating agent. The aqueous reaction solution so prepared is incubated at a temperature at which hybridization can occur and then assayed for the presence of double stranded nucleic acid molecules.

Additionally, alternative methods of the present invention are disclosed wherein the aqueous reaction solution also contains a known concentration of a nucleic acid denaturing agent and also where the nucleic acid precipitating agent is contained in a second solution which is added to the aqueous reaction solution before the incubation step.

The nucleic acid precipitating agents which are utilized to practice the various alternative methods of the present invention are preferably selected from the group consisting of detergent, dihydroxybenzene, Sarkosyl, and the alkali metal salts and ammonium salts of S0₄, P0₄, Cl, and HCOO. The salt concentrations preferably range from about 1M to about 10M. Additionally, it is preferred that the aqueous reaction solutions are prepared to have a pH ranging from about 4 to 11 and the concentration of the organic compound strong nucleic acid precipitating agents preferably ranges from approximately 5 volume % to 95 volume % and the preferred concentration of the nucleic acid denaturing agents ranges from approximately 5 volume % to 95 volume %.

Incubation temperatures preferably range from just below the Tₘ of the double stranded nucleic acid association product to temperatures approaching room temperature of approximately 22°C. It will be appreciated that the addition of nucleic acid denaturing agents to the aqueous reaction solution will lower the temperature at which hybridization occurs. The hybridization temperatures for most reactions utilizing the methods of the present invention will range from approximatley room temperature to 90°C.

After incubation, the reaction solution is assayed through a variety of known assay techniques to detect the presence of the double-stranded nucleic acid product. A preferred assay procedure utilizes hydroxyapatite for this purpose.

Further objects, features and advantages of the method of the present invention will become apparent to those skilled in the art from a consideration of the following detailed description.

### DETAILED DESCRIPTION OF THE INVENTION

In a broad aspect, the method of the present invention is based upon the surprising discovery that relatively high concentrations of nucleic acid precipitating agents (both salts and organic compounds) will greatly accelerate the rate at which single stranded nucleic acid molecules with regions of complementary base sequences will combine to form base paired double stranded nucleic acid molecules. Reaction rates are increased as much as 800 times over the standard reference condition rate for DNA:DNA reactions and as much as 3000 times over the standard reference condition rate for RNA:DNA reactions and as much as 1000 times over the standard reference condition rate for RNA:RNA reactions. What is more, these greatly increased reaction rates occur in a one-phase system and no emulsion or shaking is required.

Such significant increases in the rate of these reactions comes in complete contrast to the teachings of the prior art. For example, an often used accelerated reaction condition is approximately 1M NaCl (or an equivalent to it) which produces a reaction rate approximately 8 to 25 times faster than the reference standard condition. Such a rate increase is not the "greatly accelerated" rate disclosed by the present invention. As shown by the prior art table discussed above, increasing the concentration of NaCl to 4.75M results in little more of a rate increase. Similarly, concentrations of CsCl (also commonly used to promote increased reaction rates) up to 7.5M produce analogous increases of approximately 15 times that of the standard reference condition rate. Similar rate increases over the standard reference condition rates were also found for 1M (NH₄)₂SO₄ and 1M LiCl, each salt producing rate increases of approximately 13 fold to 18 fold, which is roughly comparable to the rate increase observed for 1M NaCl or CsCl. However, in contrast to these known results, it was surprisingly discovered that increasing the concentration of (NH₄)₂SO₄ to 2M greatly increased the reaction rate by an additional factor of 33 to approximately 600 times that of the standard reference condition rate. Additional research disclosed a comparable rate increase when the concentration of LiCl was increased to 4M wherein a rate of an additional 30 fold was observed, producing an accelerated reaction rate 467 times greater than that of the standard reference condition. Comparable patterns of great acceleration were also discovered for ammonium formate, cesium sulfate, sodium sulfate, lithium sulfate, sodium phosphate and several detergents and organic compounds.

It should be noted that other researchers (Orosz and Wetmur) have used similar salt concentrations and have not observed these greatly accelerated rate increases. It is proposed that the previous researchers did not obtain a significant rate increase as disclosed by the present invention because the salt concentration used in each specific process was too high or too low, and the concentration of single stranded material used was so high that a 100-fold increase in reaction rate was not possible. The prior art techniques generally relied upon optical methods to measure the reaction kinetics. These optical methods rely on the fact that single stranded nucleic acid absorbs more UV light than does double stranded nucleic acid. The assay is accomplished by following the decrease in optical density as the single stranded nucleic acid converts to the double stranded form. However, a large number of artifacts can occur which will interfere with these optical measurements. This is particularly true in a system where the nucleic acid has a tendency to aggregate or precipitate, as it would in the presence of these nucleic acid precipitating agents.

These same factors (aggregation or precipitation) which prevented the earlier researchers from discovering the method of the present invention are also proposed as being responsible for the greatly accelerated reaction rates of the methods of the present invention. It is hypothesized that nucleic acid precipitating agents cause the single stranded nucleic acid molecules to aggregate and thereby stimulate the reaction rate. As discussed above, the rate at which a given pair of complementary single stranded nucleic acids will form double stranded nucleic acid molecules is directly related to their concentration in the reaction solution. The higher the nucleic acid concentration, the faster the rate of reaction. In the presence of a nucleic acid precipitating agent, the single stranded nucleic acid molecules aggregate or associate together in solution. This aggregation or semi-precipitation results in high concentrations of nucleic acid in localized regions of the reaction solution. If the aggregation occurs at a temperature where hybridization or reassociation can occur, the rate of the reaction is greatly increased.

In order for the complementary single stranded nucleic acid molecules to reassociate or hybridize together in a reaction solution containing a nucleic acid precipitating agent, the temperature must be high enough for the reaction to occur. Renaturation, reassociation or hybridization usually occurs at optimal rates at roughly 10°C to 30°C below the Tₘ of the double stranded nucleic acid molecule involved. In the reaction solution of the present invention, the Tₘ of most double stranded nucleic acid molecules will range from 85°C to 100°C. When the reaction solution of the present invention contains one or more nucleic acid denaturing agents, the Tₘ will be greatly lowered and temperatures as low as room temperature can be utilized to achieve optimum reaction rates. Accordingly, reaction temperatures of approximately 20°C to 90°C should produce optimum rates of reaction.

As discussed above, a variety of nucleic acid precipitating inorganic salts have been discovered to greatly increase the rate of reaction when used at sufficiently high concentrations. In general, the salts which have been effective for the method of the present invention are those which contain at least one of the stronger salting out cation or anion groups (namely SO₄, PO₄, Li, NH₄). Additionally, organic compounds which are miscible with the reaction solution and which have precipitating or salting out properties are also effective in promoting greatly accelerated reaction rates. Examples of such compounds include detergent, dihydroxybenzene, and Sarkosyl.

To determine which salts or other compounds possess the requisite nucleic acid precipitating properties to practice the method of the present invention, it is necessary to first screen the compounds to determine if the compounds will precipitate single stranded nucleic acid molecules and then to determine whether the precipitate so formed will disappear when the reaction solution is heated to a temperature where reassociation can occur. The nucleic acid precipitating agents are then analyzed to determine the preferred concentrations and incubation temperatures for producing optimal reaction rate increases. This screening procedure also makes it possible to determine the effective concentration of the nucleic acid precipitating agent necessary to promote greatly increased reaction rates.

For example, Sarkosyl (N-lauroylsarcosine sodium salt) was screened for its ability to increase nucleic acid reassociation rates in the following manner. First, a series of solutions containing known amounts of purified liver RNA (final concentration at 4 mg/ml) and varying amounts of Sarkosyl (ranging from approximately 9 volume % to 24 volume %) were prepared. The solutions were thoroughly mixed and checked for the presence of a precipitate using either direct visual observation or a spectrophotometer using a wavelength at which neither the Sarkosyl or the nucleic acid absorbs. If a precipitate was observed in a solution, the solution was heated to approximately 40°C to 90°C to determine whether the degree of precipitation would change. It was found that at 10% to 14% Sarkosyl, little or no precipitation of the nucleic acid was observed. However, at higher concentrations, Sarkosyl was found to precipitate nucleic acid. A number of further experiments were then conducted to determine the preferred concentration and incubation temperatures for producing optimal reaction rate increases.

The following tables are an illuatrative listing of the reaction rate increases that can be expected with a variety of concentrations of preferred inorganic salt nucleic acid precipitating agents.

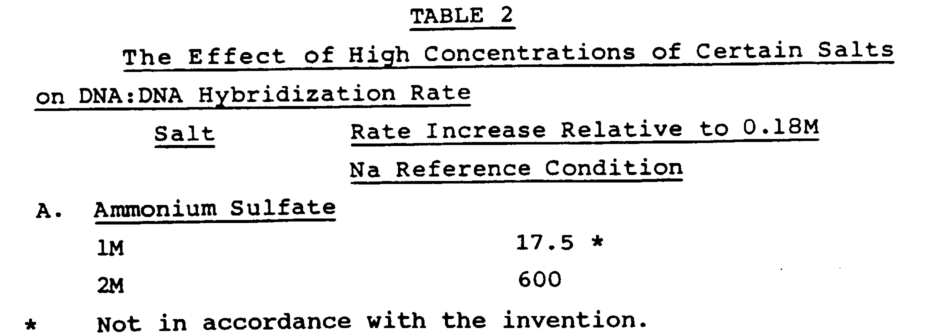

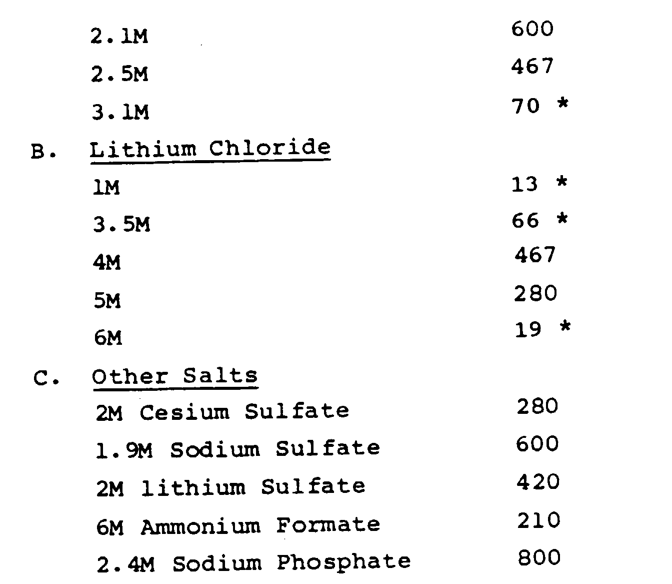

**TABLE 3**

| Effect of High Concentrations of Certain Salts on Excess RNA:DNA Hybridization Rates | |
|---|---|
| Salt | Rate of increase relative to reference 0.18M Na condition |
| 0.18M Sodium Chloride | 1 * |
| 0.72M Sodium Chloride | 3.6 * |
| 2M Ammonium Sulfate | 1500 |
| 2.4M Sodium Phosphate | 3000 |
| 4M Lithium Chloride | 600 |
| 2M Sodium Sulfate | 3460 |
| Ammonium Sulfate | |
| 1M | 90 * |
| 2M | 1500 |
| 3M | 600 |

| | |
|---|---|
| * Not in accordance with the invention. | |

With this understanding of the nucleic acid precipitating agents, the method of the present invention is as follows. The first step of the preferred method is the preparation of an aqueous reaction solution containing a quantity of a first single stranded nucleic acid molecule and a quantity of a second single stranded nucleic acid molecule, preferably incorporating a detectable marker and at least one segment of base sequences which are complementary to a corresponding segment of base sequences of the first single stranded nucleic acid molecule. Additionally, a known concentration of at least one of the previously discussed nucleic acid precipitating agents is also incorporated into the aqueous reaction solution in a concentration sufficient to greatly accelerate the rate of reaction by a factor of at least 100 times the rate of the standard reference condition reaction. While it should be emphasized that the only additional ingredient necessary to obtain the greatly increased reaction rates is the nucleic acid precipitating agent, additional additives may be incorporated into the aqueous reaction solution such as buffers, EDTA, SDS, SK, PK, or ETOH. Additionally, it should be noted that while it is preferred that at least one of the single stranded nucleic acid molecule reactants incorporates a detectable marker, the marker is not essential to promoting the greatly accelerated reaction rates.

The next step of the method of the present invention is to incubate the aqueous reaction solution. As discussed above, temperatures ranging from just below the Tₘ to approximately room temperature are sufficient for incubating the reaction solution. The actual temperatures utilized will vary depending on the concentrations of the reactants and whatever additional additives are incorporated into the reaction solution. However, most reactions will be conducted at incubation temperatures ranging from approximately room temperature to 90°C.

The last step in the method of the present invention is to assay the incubated aqueous reaction solution for the presence of double stranded nucleic acid molecules. A wide variety of assaying techniques are known in the art and are contemplated as being within the scope of the present invention. A preferred assaying technique involves the removal of an aliquot from the incubated reaction solution at a specified time after the start of the reaction. The aliquot is diluted into 1 ml of 0.14M PB, 0.02% sodium dodecyl sulfate (SDS). The diluted solution is then passed over a column of hydroxyapatite (HA) (bed volume equalling 1 ml) which has been preequilibrated to 0.14M PB, 0.02% SDS at 67°C. Single stranded DNA molecules will not bind to the HA, but RNA and double stranded nucleic acid molecules will be adsorbed to the column. Nonhybridized single stranded nucleic acid molecules are then removed from the column by passing 5 ml of column buffer 0.14M PB, 0.02% SDS over the column. The adsorbed nucleic acid is recovered from the column by eluting the column with 0.3M PB at 67°C. The various solution fractions so produced may then be assayed for the detectable marker (such as radioactive hydrogen).

An alternative approach for practicing the method of the present invention incorporates the additional step of mixing a second solution containing the nucleic acid precipitating agent into the previously prepared aqueous reaction solution prior to incubating the resultant mixture. Thus, the alternative method comprises the steps of preparing the previously discussed aqueous reaction solution, mixing the aqueous reaction solution with a second solution containing a known concentration of at least one nucleic acid precipitating agent which is miscible with the aqueous solution and capable of precipitating single stranded nucleic acid molecules from an aqueous solution, incubating the resulting mixture at the previously discussed temperatures, and assaying the incubated mixture for the presence of double stranded nucleic acid molecules. This alternative method serves to eliminate any problems which may occur with the premature aggregation of the single stranded nucleic acid molecule reactants in the aqueous reaction solution.

An additional modification to both of the alternative methods for practicing the accelerated rate reaction of the present invention involves the addition of a known concentration of at least one nucleic acid denaturing agent such as alcohol to the aqueous reaction solution. Preferably the concentration of denaturing agent added will range from approximately 5% by volume to approximately 95% by volume. For example, ethanol is a denaturant and functions to lower the temperature at which the reaction will occur. Ethanol is soluble in 2M (NH₄)₂SO₄ to approximately 20%. At this concentration, the reaction will occur at a temperature of approximately 49°C instead of the usual 60° to 80°C.

Regardless of which of the alternative methods is utilized to practice the method of the present invention, the nucleic acid precipitating agents are preferably selected from the group consisting of detergent, dihydroxybenzene, Sarkosyl, and the alkali metal salts and ammonium salts of SO₄, PO₄, Cl, and HCOO. It is also contemplated as being within the scope of the present invention to combine various members of this group in a single aqueous reaction solution. Additionally, it is also contemplated as being within the scope of the present invention to utilize a variety of detergent agents in addition to the organic compounds disclosed. Accordingly, those compounds specifically disclosed in the present invention are those which are currently known to be suitable for practicing the method of the present invention. Use of analogous compounds is therefore considered to be within the scope of the present invention.

It will be appreciated that the effective concentrations of nucleic acid precipitating agents necessary to practice the method of the present invention will vary with the amount of nucleic acid in the reaction solution and the pH of the solution as well as with the presence of other compounds. Accordingly, it is preferred that the concentrations will range from approximatley 1M to 10M for the inorganic salt compounds and from approximately 5% by volume to approximatley 95% by volume for the organic compounds. Additionally, it is preferred that the pH of the reaction solution will range from approximately 4 to 11.

Lastly, as discussed above, the preferred incubation temperature for the aqueous reaction solution should range from approximately room temperature to approximately 90°C.

The method of the present invention is suitable for bacterial, viral, mammalian and chemically synthesized nucleic acid. The completeness of the reaction will vary depending upon the concentration of the strong nucleic acid precipitating agent as well as on the amount of nucleic acid in the original reaction solution. At low concentrations of nucleic acid, well over 90% of the single stranded nucleic acid will associate to form double stranded nucleic acid molecules. At higher nucleic acid concentrations, the completeness of the reaction will only be approximately 70% or less even though the rate of the reaction will be greatly increased. It should be noted that at very high concentrations of nucleic acid reactants, the rate of reaction will be accelerated to a lesser degree.

The amount of single stranded nucleic acid molecule reactants present in the aqueous reaction solution can range from an upward extreme in which the second single stranded nucleic acid molecule is present in an initial concentration such that at least a 100 fold increase in acceleration of rate of reaction is observed in the presence of 4M LiCl compared with the rate or reaction observed with 0.18 M NaCl at 60°C, to a lower extreme on the order of 10⁻⁹ micrograms. Interestingly, the reaction rate increase for high concentrations of DNA:DNA or DNA:RNA reactions is lower than that for low concentrations of DNA:DNA and RNA:RNA reactions. Thus, the method of the present invention is applicable to both high and low concentrations of reactants. Along these lines, preferred reaction solution volumes will be on the order of a millilitre or less to a fraction of a microlitre. However, it should be emphasized that other reaction solution volumes are contemplated as being within the scope of the present invention. Additionally, while the presence of small quantities of protein and other cell components will not greatly interfere with the reaction of the method of the present invention, excess heterologous RNA or DNA will slow the reaction rate to various extents. It should also be noted that the greatly accelerated reaction rates have been achieved for nucleic acid molecules ranging from approximately 60 bases long to molecules on the order of 10⁴ bases long. However, the method of the present invention is contemplated as being applicable to nucleic acid molecules ranging from short molecules on the order of 10 to 15 bases long to longer molecules in excess of 10⁴ bases long.

The following examples are offered as being illustrative of the method of the present invention and not by way of limitation.

### EXAMPLE 1

### Method for Using Sodium Phosphate to Increase DNA:DNA Hybridization Rates

1. Mix throroughly:
   50 microliters of 0.17% SDS, 3 x 10⁻³M EDTA, containing 0.004 mcg of sonicated single strand ³H - E. Coli. DNA of about 300 to 700 bases in length.
   + 50 microliters of 4.8M sodium phosphate pH = 6.8
2. Incubate the mixture at 76°C and remove aliquots at specified times after the start. Dilute the aliquots into 0.14M PB 0.02% SDS and assay for hybridization using hydroxyapatite as described earlier.

The above procedure results in a rate increase of about 800 relative to the rate at the standard reference condition.

Such large rate increases can be attained with a variety of different volumes, concentrations of sodium phosphate, DNA concentrations, EDTA and SDS concentrations and temperatures of incubation.

### EXAMPLE 2

### Method for Using Sodium Sulfate to Increase DNA:DNA Rates

1. Mix thoroughly:
   0.15 ml water containing 2 mcg of ³H - E. Coli sonicated single strand DNA (300 to 700 bases long)
   + 0.85 ml of 2.25M Sodium sulfate.
2. Incubate the mixture at 77°C and remove aliquots at specified times after the start. Dilute the aliquot into 1 ml 0.14M PB, 0.02% SDS and assay on HA as described earlier.

This procedure results in a rate increase of about 600 fold relative to the reference condition.

### EXAMPLE 3

### LiCl Rate Increase Method for DNA:DNA Hybridization

1. Mix thoroughly:
   0.3ml 0.16M Tris pH=7.8, containing about 10 mcg sonicated single strand ³H - E. Coli DNA (about 300 to 700 bases long)
   + 0.2 ml 10M lithium chloride.
2. Incubate the mixture at 76°C and remove aliquots at specified times after the start. Dilute the aliquot and assay for hybridization as described earlier.

This procedure results in a rate increase of about 600 relative to the reference condition.

### EXAMPLE 4

Ammonium Sulfate Method for DNA:DNA Hybridization Rate Increase

### A. Bacterial DNA

1. Mix thoroughly:
   50 microliters of 0.2 Tris pH = 7.8, containing about 5 mcg of sonicated single strand ³H - E. Coli DNA (about 300 to 700 bases long);
   + 50 microliters 4.OM Ammonium sulfate.
2. Incubate the mixture at 78°C and remove aliquots at specified times after the start. Dilute the aliquot and assay for hybridization as described earlier.

This procedure resulted in a rate increase of about 700 relative to the reference condition.

### EXAMPLE 5

### B. Bacterial DNA: Ethanol Modification

The temperature at which the hybridization is conducted can be lowered dramatically by adding ethanol to the reaction mixture. Ethanol is soluble to about 20% in 2M Ammonium sulfate.
1. Mix thoroughly:
   0.05 ml of 34% ethanol in water containing 0.4 mcg of sonicated single strand ³H - E. Coli DNA (300 to 700 bases long).
   + 0.05 ml 4M ammonium sulfate, 0.01M EDTA, 0.1M PB ph = 6.8
2. Incubate the mixture at 49°C for appropriate times and remove aliquots. Dilute and assay the aliquots for hybridization as described earlier.

This procedure results in a rate increase of about 100 fold relative to the reference condition.

### EXAMPLE 6

### C. Mammalian DNA: Low Concentration

1. Mix thoroughly:
   0.1 ml of 0.02M EDTA containing 26 mcg of sonicated single strand ³H human DNA (about 400 to 800 bases long):
   + 0.1 ml 4M Ammonium sulfate, 0.1M PB pH = 6.8
2. Incubate the mixture at 68°C and at specified times remove aliquots. Dilute each aliquot and assay for hybridization as described earlier.

This procedure results in a rate increase of about 200 fold relative to the reference condition.

### EXAMPLE 7

D. Mammalian DNA: Low concentration: Ethanol Modification
1. Mix thoroughly:
   0.05 ml of 0.016M EDTA, 40% ethanol in water containing 26 mcg sonicated single strand ³H human DNA (about 400 to 800 bases long);
   + 0.05 ml 4M ammonium sulfate, 0.1M PB pH = 6.8
2. Incubate the mixture at 49°C and at specified times remove aliquots. Dilute each aliquot and assay it for hybridization as described earlier.

This procedure resulted in a rate increase of about 180 fold relative to the reference condition.

### EXAMPLE 8 (Comparative)

### E. Mammalian DNA: High Concentration

1. Mix thoroughly:
   0.025 ml of 0.04M EDTA in water containing 130 mcg of sonicated single strand human ³H DNA (about 400 to 800 bases long);
   + 0.025 ml 4M ammonium sulfate, 0.1M PB pH = 6.8
2. Incubate the mixture at 68°C and at specified times remove aliquots. Dilute each aliquot and assay for hybridization as described earlier.

This procedure results in a rate increase of about 90 fold relative to the reference condition.

### EXAMPLE 9

### F. Mammalian DNA: High Concentration: Ethanol Modification

1. Mix thoroughly:
   0.0125 ml of 0.016M EDTA in water containing 40% ETOH and 65 mcg of sonicated single strand human ³H DNA (400 to 800 bases long);
   + 0.0125 ml 4M ammonium sulfate, 0.1M PB pH = 6.8
2. Incubate the mixture at 49°C and at specified times remove aliquots. Dilute each aliquot and assay it for hybridization as described earlier.

This procedure results in a rate increase of about 130 fold relative to the reference condition.

### EXAMPLE 10

### Purified RNA:DNA Hybridization Rate Increases with Sodium Phosphate

### A. Excess RNA: 0.2 ml Volume

1. Thoroughly mix:
   0.1 ml of 0.2% SDS, 10⁻³M EDTA in water containing 2X10⁻³ mcg of Polio I RNA and 2x10⁻⁴ mcg of ³H-cDNA (³H-DNA complementary to Polio RNA, 300 to 600 bases long)
   + 0.1 ml 4.8M sodium phosphate pH = 6.8
2. Incubate the mixture at 76 C and at specified times remove aliquots. Dilute each aliquot and assay it for hybridization as described earlier.

This procedure results in a hybridization rate increase of 3300 fold over the rate in the standard reference condition of 60°C, 0.18M Na.

### EXAMPLE 11

### B. Excess MA: 1 ml volume

1. Thoroughly mix:
   0.5 ml 10⁻³M EDTA, 0.2% SDS containing 2X10⁻³ mcg Polio I RNA and 2X10⁻⁴ mcg of 300 to 600 base long Polio ³H-CDNA;
   + 0.5 ml 4.8M sodium phosphate pH = 6.8
2. Incubate at 76°C and at specified times remove aliquots. Dilute each aliquot and assay it for hybridization as described earlier.

This procedure results in a hybridization rate increase of 3300 fold over the reference condition rate.

### EXAMPLE 12

### C. Excess RNA: Plus Added Heterologous High Molecular Weight RNA

1. Thoroughly mix:
   0.05 ml 10⁻³M EDTA, 0.4% SDS in water containing 2x10⁻³ mcg Polio I RNA, 2x10⁻⁴ mcg Polio I ³H-cDNA (300 to 600 bases long) and 5 mcg. of calf liver RNA;
   + 0.05 ml 5.1M sodium phosphate.
2. Incubate the mixture at 76°C and at specified times remove aliquots. Dilute each aliquot and assay it for hybridization as described earlier.

This procedure resulted in a rate increase of 600 fold over the rate at the reference condition.

### EXAMPLE 13

### D. Excess DNA:

1. Thoroughly mix:
   0.012 ml 0.012% Sarkosyl containing 1.5 x 10-6 mcg of ³H-cDNA and 1.2 x 10-6 ribosomal RNA from Legionella penumophilia. The ³H cDNA (100 to 300 bases long) is complementary to only about one third of the RNA. The cDNA/RNA ratio is about 4/1 for the complementary RNA and DNA sequences.
   + 0.02 ml of 4.8M sodium phosphate pH = 6.8
2. Incubate the mixture at 76°C and at specified times remove aliquots. Dilute each aliquot and assay it for hybridization.

This procedure results in a rate increase of greater than 3000 over the rate at the reference condition.

### EXAMPLE 14

### E. Excess DNA: Non Purified RNA

1. Thoroughly mix:
   0.012 ml of 1.4x10⁻³M EDTA, 1.4x10⁻³M EGTA, 0.7% SDS, 0.3% Sarkosyl containing 10 mcg Proteinase K, 10-4 mcg of ³H-cDNA (100 to 300 bases long) complementary to Legionella pneumophila ribosomal RNA and 4400 Legionella pneumophila bacteria which contain about 6x10⁻⁵ mcg. of ribosomal RNA.
   +0.02 ml of 4.8M sodium phosphate pH = 6.8
2. Incubate the mixture at 76°C and at specified times remove aliquots. Dilute each aliquot and assay each aliquot for hybridization as described earlier.

This procedure results in a rate increase of greater than 150 fold over the rate in the reference condition.

### EXAMPLE 15

### F. Excess RNA: Non Purified RNA

1. Thoroughly mix:
   0.012 ml of 0.16% Sarkosyl containing 10⁻⁵ mcg of ³H-cDNA (100 to 300 bases long) complementary to E. Coli ribosomal RNA and 5000 E. Coli bacteria which contain about 7x10⁻⁵ mcg ribosomal RNA
   +0.02 ml of 4.8M sodium phosphate pH = 6.8
2. Incubate the mixture at 76°C and at specified times remove aliquots. Dilute each aliquot and assay it for hybridization as described earlier.

This procedure results in a rate increase of greater than 100 fold over the rate at the reference condition.

### EXAMPLE 16

### RNA:DNA Hybridization Rate Increase Promoted by Sodium Sulfate

1. Mix well;
   0.023 ml of 10⁻³M EDTA, 0.1% SDS containing 2x10⁻³ mcg Polio I RNA and 2x10⁻⁴ mcg ³H-cDNA (300 to 600 bases long) which is complementary to Polio I RNA.
   + 0.178 ml 2.25M sodium sulfate.
2. Incubate at 76°C and at specified times remove aliquots. Dilute aliquot and assay it for hybridization as described earlier.

This procedure resulted in a rate increase of over 3000 relative to the reference rate.

### EXAMPLE 17

RNA:DNA Hybridization Rate Increase Promoted by Ammonium Sulfate
1. Mix well;
   0.1 ml of 0.2% SDS, 10⁻³M EDTA containing 2x10⁻³ mcg of Polio I RNA and 2x10⁻⁴ mcg of ³H=cDNA (300 to 600 bases long) which is complementary to Polio I RNA
   + 0.1 ml of 4M ammonium sulfate.
2. Incubate at 77°C and at specified times remove aliquots. Dilute each aliquot and assay it for hybridization as described earlier.

This procedure resulted in a rate increase of about 1500 relative to the reference conditon rate.

### EXAMPLE 18

RNA:RNA Hybridization Rate Increase Promoted by Ammonium Sulfate
1. Mix well;
   0.1 ml 0.2% SDS, 10⁻³M EDTA containing 4x10⁻³ mcg VSV RNA and 2x10⁻⁴ mcg ¹²⁵I-cRNA (about 300 to 800 bases long) which is complementary to VSV RNA.
   + 0.1 ml 4.4M ammonium sulfate.
2. Incubate at 87°C and at specified times remove aliquots. Dilute each aliquot and assay it for hybridization by a standard ribonuclease technique.

This procedure resulted in a rate increase of over 1000 relative to the 0.18M Na rate.

### EXAMPLE 19

### RNA:RNA Hybridization Rate Increase Promoted by Sodium Phosphate

1. Mix well;
   0.1 ml 0.2% SDS, 10⁻³M EDTA containing 4x10⁻³ mcg VSV RNA and 2.10⁻⁴ mcg VSV ¹²⁵I-cRNA which is complementary to VSV RNA.
   + 0.1 ml 4.8M sodium phosphate.
2. Incubate at 83°C and at specified times remove aliquots. Dilute each aliquot and assay it for hybridization by a standard ribonuclease technique.

This procedure results in a rate increase of greater than 500 relative to the reference condition rate.

In closing, it should be understood that the embodiments of the present invention disclosed herein are illustrative of the principles of the invention and that other modifications, nucleic acid precipitating agents or nucleic acid denaturing agents may be employed which are within the scope of the invention. However, the methods disclosed and described herein are preferred. Accordingly, the present invention is not limited to that precisely as disclosed and described.

## Claims (Claims for the following Contracting State(s): AT, BE, CH, DE, FR, GB, LI, LU, NL, SE)

1. An improved method for the formation of double-stranded nucleic acid molecules from separate single-stranded nucleic acid molecules wherein the rate of reaction is greatly increased over the standard reference condition reaction rate, said method comprising the steps of:
- preparing an aqueous reaction solution containing a quantity of a first single-stranded nucleic acid molecule,
- a quantity of a second single-stranded nucleic acid molecule, said second single-stranded nucleic acid molecule having at least one segment of base sequences complementary to a corresponding segment of base sequences of said first single-stranded nucleic acid molecule, the concentration of the second nucleic acid being such that at least a 100 fold increase in acceleration of rate of reaction is observed in the presence of 4 M LiCl compared with the rate of reaction observed with 0.18 M NaCl at 60°C; and
- at least one nucleic acid precipitating agent other than sodium phosphate or sodium sarcosinate, which precipitating agent is miscible with an aqueous solution and capable of precipitating single stranded nucleic acids from an aqueous solution, in a concentration sufficient to accelerate the rate of reaction of the said first and second single-stranded nucleic acid molecules at least about 100 times compared with the rate of reaction in solution using 0.18 M NaCl at 60°C;
incubating said aqueous reaction solution at a temperature at which reassociation can occur; and
assaying said incubated aqueous reaction solution for the presence of double-stranded nucleic acid molecules.

2. The method of claim 1 wherein said nucleic acid precipitating agent is selected from detergent, dihydroxybenzene, Sarkosyl, and the alkali metal salts and ammonium salts of SO₄, PO₄, C1, and HCOO.

3. The method of claim 1 or 2 wherein said nucleic acid precipitating agent is provided in a concentration ranging from about 1M to about 10M.

4. The method of any one of the preceding claims wherein said aqueous reaction solution also contains a known concentration of a nucleic acid denaturing agent.

5. The method of claim 4 wherein said known concentration of nucleic acid denaturing agent ranges from about 5% volume to about 95% by volume.

6. The method of claim 4 wherein said nucleic acid denaturing agent is alcohol ranging in concentration from about 10% by volume to 20% by volume and said nucleic acid precipitating agent is (NH₄)₂SO₄.

7. The method of any one of the preceding claims wherein the pH of said aqueous reaction solution ranges from about 4 to about 11.

8. The method of any one of the preceding claims wherein said temperature ranges from about 20°C to about 90°C.

9. The method of any one of claims 1 to 5, 7 or 8 wherein said nucleic acid precipitating agent is NaSO₄.

10. The method of any one of claims 1 to 5, 7 or 8 wherein said nucleic acid precipitating agent is LiCl.

11. The method of any one of claims 1 to 5, 7 or 8 wherein said nucleic acid precipitating agent is (NH₄)₂SO₄.

12. The method of any one of claims 1, 2, 4, 7 or 8 wherein said nucleic acid precipitating agent is 13 volume % to 30 volume % Sarkosyl.

13. The method of claim 1 wherein an aqueous reaction solution containing the first single-stranded nucleic acid molecule and the second single-stranded nucleic acid molecule is mixed with a solution containing the nucleic acid precipitating agent.

14. An assay method according to any one of claims 1 to 13 wherein said second nucleic acid molecule is present in a clinical sample and detecting whether said second nucleic acid molecule is present in said sample.

15. A method for screening a suspected nucleic acid precipitating agent for use in an accelerated nucleic acid reassociation reaction, wherein the rate of the reassociation reaction is at least about 100 times the rate of reaction in solution using 0.18 M NaCl at 60°C, said screening method comprising the steps of:
preparing a series of solutions, each of said solutions containing a known amount of single-stranded nucleic acid and a different amount of said suspected nucleic acid precipitating agent;
examining each of said solutions for the presence of a nucleic acid precipitate; and
heating each of said solutions in which said nucleic acid precipitate was found in order to determine whether the degree of precipitation changes.

## Claims (Claims for the following Contracting State(s): IT)

1. An improved method for the formation of double-stranded nucleic acid molecules from separate single-stranded nucleic acid molecules wherein the rate of reaction is greatly increased over the standard reference condition reaction rate, said method comprising the steps of:
- preparing an aqueous reaction solution containing a quantity of a first single-stranded nucleic acid molecule,
- a quantity of a second single-stranded nucleic acid molecule, said second single-stranded nucleic acid molecule having at least one segment of base sequences complementary to a corresponding segment of base sequences of said first single-stranded nucleic acid molecule, the concentration of the second nucleic acid being such that at least a 100 fold increase in acceleration of rate of reaction is observed in the presence of 4 M LiCl compared with the rate of reaction observed with 0.18 M NaCl at 60°C; and
- at least one nucleic acid precipitating agent which is miscible with an aqueous solution and capable of precipitating single stranded nucleic acids from an aqueous solution, in a concentration sufficient to accelerate the rate of reaction of the said first and second single-stranded nucleic acid molecules at least about 100 times compared with the rate of reaction in solution using 0.18 M NaCl at 60°C;
incubating said aqueous reaction solution at a temperature at which reassociation can occur; and
assaying said incubated aqueous reaction solution for the presence of double-stranded nucleic acid molecules.

2. The method of claim 1 wherein said nucleic acid precipitating agent is selected from detergent, dihydroxybenzene, Sarkosyl, and the alkali metal salts and ammonium salts of SO₄, PO₄, C1, and HCOO.

3. The method of claim 1 or 2 wherein said nucleic acid precipitating agent is provided in a concentration ranging from about 1M to about 10M.

4. The method of any one of the preceding claims wherein said aqueous reaction solution also contains a known concentration of a nucleic acid denaturing agent.

5. The method of claim 4 wherein said known concentration of nucleic acid denaturing agent ranges from about 5% volume to about 95% by volume.

6. The method of claim 4 wherein said nucleic acid denaturing agent is alcohol ranging in concentration from about 10% by volume to 20% by volume and said nucleic acid precipitating agent is (NH₄)₂SO₄.

7. The method of any one of the preceding claims wherein the pH of said aqueous reaction solution ranges from about 4 to about 11.

8. The method of any one of the preceding claims wherein said temperature ranges from about 20°C to about 90°C.

9. The method of any one of claims 1 to 5, 7 or 8 wherein said nucleic acid precipitating agent is NaSO₄.

10. The method of any one of claims 1 to 5, 7 or 8 wherein said nucleic acid precipitating agent is LiCl.

11. The method of any one of claims 1 to 5, 7 or 8 wherein said nucleic acid precipitating agent is (NH₄)₂SO₄.

12. The method of any one of claims 1, 2, 4, 7 or 8 wherein said nucleic acid precipitating agent is 13 volume % to 30 volume % Sarkosyl.

13. The method of claim 1 wherein an aqueous reaction solution containing the first single-stranded nucleic acid molecule and the second single-stranded nucleic acid molecule is mixed with a solution containing the nucleic acid precipitating agent.

14. An assay method according to any one of claims 1 to 13 wherein said second nucleic acid molecule is present in a clinical sample and detecting whether said second nucleic acid molecule is present in said sample.

15. A method for screening a suspected nucleic acid precipitating agent for use in an accelerated nucleic acid reassociation reaction, wherein the rate of the reassociation reaction is at least about 100 times the rate of reaction in solution using 0.18 M NaCl at 60°C, said screening method comprising the steps of:
preparing a series of solutions, each of said solutions containing a known amount of single-stranded nucleic acid and a different amount of said suspected nucleic acid precipitating agent;
examining each of said solutions for the presence of a nucleic acid precipitate; and
heating each of said solutions in which said nucleic acid precipitate was found in order to determine whether the degree of precipitation changes.

## Revendications (Revendications pour l'(les) Etat(s) contractant(s) suivant(s): AT, BE, CH, DE, FR, GB, LI, LU, NL, SE)

1. Méthode améliorée pour la formation de molécules d'acide nucléique double brin à partir de molécules d'acide nucléique simple brin séparées dans laquelle la vitesse de réaction est fortement augmentée par rapport à la vitesse de réaction dans des conditions de référence standard, ladite méthode comprenant les étapes consistant à :
- préparer une solution réactionnelle aqueuse contenant une quantité d'une première molécule d'acide nucléique simple brin,
- une quantité d'une seconde molécule d'acide nucléique simple brin, ladite seconde molécule d'acide nucléique simple brin ayant au moins un segment de séquences de bases complémentaire d'un segment correspondant de séquences de bases de ladite première molécule d'acide nucléique simple brin, la concentration du second acide nucléique étant telle que l'on observe au moins une augmentation de 100 fois de l'accélération de la vitesse de réaction en présence de LiCl 4 M en comparaison avec la vitesse de réaction observée avec NaCl 0,18 M à 60°C ; et
- au moins un agent de précipitation des acides nucléiques autre que le phosphate de sodium ou le sarcosinate de sodium, agent de précipitation qui est miscible avec une solution aqueuse et est capable de provoquer la précipitation des acides nucléiques simple brin à partir d'une solution aqueuse, en une concentration suffisante pour accélérer la vitesse de réaction desdites première et seconde molécules d'acide nucléique simple brin au moins environ 100 fois en comparaison avec la vitesse de réaction en solution en utilisant NaCl 0,18 M à 60°C ;
incuber ladite solution réactionnelle aqueuse à une température à laquelle une réassociation peut se produire ; et
rechercher dans ladite solution réactionnelle aqueuse incubée la présence de molécules d'acide nucléique double brin.

2. Méthode de la revendication 1 dans laquelle ledit agent de précipitation des acides nucléiques est choisi parmi un détergent, du dihydroxybenzène, du Sarkosyl, et les sels de métaux alcalins et sels d'ammonium de S0₄, PO₄, Cl et HCOO.

3. Méthode de la revendication 1 ou 2 dans laquelle ledit agent de précipitation des acides nucléiques est fourni à une concentration comprise entre environ 1 M et environ 10 M.

4. Méthode de l'une quelconque des revendications précédentes dans laquelle ladite solution réactionnelle aqueuse contient également une concentration connue d'un agent de dénaturation des acides nucléiques.

5. Méthode de la revendication 4 dans laquelle ladite concentration connue d'un agent de dénaturation des acides nucléiques est comprise entre environ 5 % en volume et environ 95 % en volume.

6. Méthode de la revendication 4 dans laquelle ledit agent de dénaturation des acides nucléiques est un alcool dont la concentration est comprise entre environ 10 % en volume et 20 % en volume et ledit agent de précipitation des acides nucléiques est (NH₄)₂SO₄.

7. Méthode de l'une quelconque des revendications précédentes dans laquelle le pH de ladite solution réactionnelle aqueuse est compris entre environ 4 et environ 11.

8. Méthode de l'une quelconque des revendications précédentes dans laquelle ladite température est comprise entre environ 20°C et environ 90°C.

9. Méthode de l'une quelconque des revendications 1 à 5, 7 ou 8 dans laquelle ledit agent de précipitation des acides nucléiques est NaSO₄.

10. Méthode de l'une quelconque des revendications 1 à 5, 7 ou 8 dans laquelle ledit agent de précipitation des acides nucléiques est LiCl.

11. Méthode de l'une quelconque des revendications 1 à 5, 7 ou 8 dans laquelle ledit agent de précipitation des acides nucléiques est (NH₄)₂SO₄.

12. Méthode de l'une quelconque des revendications 1, 2, 4, 7 ou 8 dans laquelle ledit agent de précipitation des acides nucléiques est du Sarkosyl à 13 % en volume jusqu'à 30 % en volume.

13. Méthode de la revendication 1 dans laquelle une solution réactionnelle aqueuse contenant la première molécule d'acide nucléique simple brin et la seconde molécule d'acide nucléique simple brin est mélangée avec une solution contenant l'agent de précipitation des acides nucléiques.

14. Méthode de test selon l'une quelconque des revendications 1 à 13 dans laquelle ladite seconde molécule d'acide nucléique est présente dans un échantillon clinique et consistant à détecter si ladite seconde molécule d'acide nucléique et présente dans ledit échantillon.

15. Méthode pour rechercher un agent de précipitation des acides nucléiques potentiel pour une utilisation dans une réaction de réassociation des acides nucléiques accélérée, où la vitesse de la réaction de réassociation est au moins environ 100 fois la vitesse de réaction en solution en utilisant NaCl 0,18 M à 60°C, ladite méthode de recherche comprenant les étapes consistant à :
préparer une série de solutions, chacune desdites solutions contenant une quantité connue d'acide nucléique simple brin et une quantité différente dudit agent de précipitation des acides nucléiques potentiel;
rechercher dans chacune desdites solutions la présence d'un précipité d'acide nucléique ; et
chauffer chacune desdites solutions dans lesquelles on a trouvé ledit précipité d'acide nucléique afin de déterminer si le degré de précipitation change.

## Revendications (Revendications pour l'(les) Etat(s) contractant(s) suivant(s): IT)

1. Méthode améliorée pour la formation de molécules d'acide nucléique double brin à partir de molécules d'acide nucléique simple brin séparées dans laquelle la vitesse de réaction est fortement augmentée par rapport à la vitesse de réaction dans des conditions de référence standard, ladite méthode comprenant les étapes consistant à :
- préparer une solution réactionnelle aqueuse contenant une quantité d'une première molécule d'acide nucléique simple brin,
- une quantité d'une seconde molécule d'acide nucléique simple brin, ladite seconde molécule d'acide nucléique simple brin ayant au moins un segement de séquences de bases complémentaire d'un segment correspondant de séquences de bases de ladite première molécule d'acide nucléique simple brin, la concentration du second acide nucléique étant telle que l'on observe au moins une augmentation de 100 fois de l'accélération de la vitesse de réaction en présence de LiCl 4 M en comparaison avec la vitesse de réaction observée avec NaCl 0,18 M à 60°C ; et
- au moins un agent de précipitation des acides nucléiques qui est miscible avec une solution aqueuse et est capable de provoquer la précipitation des acides nucléiques simple brin à partir d'une solution aqueuse, en une concentration suffisante pour accélérer la vitesse de réaction desdites première et seconde molécules d'acide nucléique simple brin au moins environ 100 fois en comparaison avec la vitesse de réaction en solution en utilisant NaCl 0,18 M à 60°C ;
incuber ladite solution réactionnelle aqueuse à une température à laquelle une réassociation peut se produire ; et
rechercher dans ladite solution réactionnelle aqueuse incubée la présence de molécules d'acide nucléique double brin.

2. Méthode de la revendication 1 dans laquelle ledit agent de précipitation des acides nucléiques est choisi parmi un détergent, du dihydroxybenzène, du Sarkosyl, et les sels de métaux alcalins et sels d'ammonium de SO₄, PO₄, Cl et HCOO.

3. Méthode de la revendication 1 ou 2 dans laquelle ledit agent de précipitation des acides nucléiques est fourni à une concentration comprise entre environ 1 M et environ 10 M.

4. Méthode de l'une quelconque des revendications précédentes dans laquelle ladite solution réactionnelle aqueuse contient également une concentration connue d'un agent de dénaturation des acides nucléiques.

5. Méthode de la revendication 4 dans laquelle ladite concentration connue d'un agent de dénaturation des acides nucléiques est comprise entre environ 5 % en volume et environ 95 % en volume.

6. Méthode de la revendication 4 dans laquelle ledit agent de dénaturation des acides nucléiques est un alcool dont la concentration est comprise entre environ 10 % en volume et 20 % en volume et ledit agent de précipitation des acides nucléiques est (NH₄)₂SO₄.

7. Méthode de l'une quelconque des revendications précédentes dans laquelle le pH de ladite solution réactionnelle aqueuse est compris entre environ 4 et environ 11.

8. Méthode de l'une quelconque des revendications précédentes dans laquelle ladite température est comprise entre environ 20°C et environ 90°C.

9. Méthode de l'une quelconque des revendications 1 à 5, 7 ou 8 dans laquelle ledit agent de précipitation des acides nucléiques est NaSO₄.

10. Méthode de l'une quelconque des revendications 1 à 5, 7 ou 8 dans laquelle ledit agent de précipitation des acides nucléiques est LiCl.

11. Méthode de l'une quelconque des revendications 1 à 5, 7 ou 8 dans laquelle ledit agent de précipitation des acides nucléiques est (NH₄)₂SO₄.

12. Méthode de l'une quelconque des revendications 1, 2, 4, 7 ou 8 dans laquelle ledit agent de précipitation des acides nucléiques est du Sarkosyl à 13 % en volume jusqu'à 30 % en volume.

13. Méthode de la revendication 1 dans laquelle une solution réactionnelle aqueuse contenant la première molécule d'acide nucléique simple brin et la seconde molécule d'acide nucléique simple brin est mélangée avec une solution contenant l'agent de précipitation des acides nucléiques.

14. Méthode de test selon l'une quelconque des revendications 1 à 13 dans laquelle ladite seconde molécule d'acide nucléique est présente dans un échantillon clinique et consistant à détecter si ladite seconde molécule d'acide nucléique est présente dans ledit échantillon.

15. Méthode pour rechercher un agent de précipitation des acides nucléiques potentiel pour une utilisation dans une réaction de réassociation des acides nucléiques accélérée, où la vitesse de la réaction de réassociation est au moins environ 100 fois la vitesse de réaction en solution en utilisant NaCl 0,18 M à 60°C, ladite méthode de recherche comprenant les étapes consistant à :
préparer une série de solutions, chacune desdites solutions contenant une quantité connue d'acide nucléique simple brin et une quantité différente dudit agent de précipitation des acides nucléiques potentiel ;
rechercher dans chacune desdites solutions la présence d'un précipité d'acide nucléique ; et
chauffer chacune desdites solutions dans lesquelles on a trouvé ledit précipité d'acide nucléique afin de déterminer si le degré de précipitation change.

## Patentansprüche (Patentansprüche für folgende(n) Vertragsstaat(en): AT, BE, CH, DE, FR, GB, LI, LU, NL, SE)

1. Verbessertes Verfahren zur Herstellung doppelsträngiger Nukleinsäure-Moleküle aus getrennten einzelsträngigen Nukleinsäure-Molekülen, wobei die Geschwindigkeit der Reaktion gegenüber der Reaktionsgeschwindigkeit unter Standardbezugsbedingungen stark erhöht ist, welches Verfahren die folgenden Schritte umfaßt:
Herstellen einer wässrigen Reaktionslösung, umfassend eine Quantität eines ersten einzelsträngigen Nukleinsäure-Moleküls,
- eine Quantität eines zweiten einzelsträngigen Nukleinsäure-Moleküls, welches zweite einzelsträngige Nukleinsäure-Molekül mindestens ein Segment der Basensequenzen aufweist, das komplementär zu einem entsprechenden Segment der Basensequenzen des ersten einzelsträngigen Nukleinsäure-Moleküls ist, wobei die Konzentration der zweiten Nukleinsäure eine derartige ist, daß zumindest eine 100fache Zunahme der Beschleunigung der Reaktionsrate bei Vorhandensein von 4 M LiCl, im Vergleich zur mit 0,18 M NaCl bei 60°C beobachteten Reaktionsgeschwindigkeit, beobachtet wird; und
- mindestens ein Nukleinsäure-Fällmittel, das ein anderes als Natriumphosphat oder Natriumsarcosinat ist, welches Fällmittel mit einer wässrigen Lösung mischbar und zum Ausfällen einzelsträngiger Nukleinsäuren aus einer wässrigen Lösung fähig ist, in einer ausreichenden Konzentration, um die Reaktionsgeschwindigkeit der ersten und zweiten einzelsträngigen Nukleinsäure-Moleküle mindestens etwa 100fach, im Vergleich zur Reaktionsgeschwindigkeit in Lösung unter Verwendung von 0,18 M NaCl bei 60°C, zu beschleunigen;
Inkubieren der wässrigen Reaktionslösung bei einer Temperatur, bei der die Reassoziation stattfinden kann; und
Analysieren der inkubierten wässrigen Reaktionslösung auf das Vorhandensein doppelsträngiger Nukleinsäure-Moleküle.

2. Verfahren nach Anspruch 1, wobei das Nukleinsäure-Fällmittel gewählt wird aus Reinigungsmittel, Dihydroxybenzol, Sarkosyl und den Alkalimetallsalzen und Ammoniumsalzen von SO₄, PO₄, C₁ und HCOO.

3. Verfahren nach Anspruch 1 oder 2, wobei das Nukleinsäure-Fällmittel in einer Konzentration im Bereich von etwa 1 M bis etwa 10 M bereitgestellt wird.

4. Verfahren nach einem der vorhergehenden Ansprüche, wobei die wässrige Reaktionslösung außerdem eine bekannte Konzentration eines Nukleinsäure-Denaturierungsmittels enthält.

5. Verfahren nach Anspruch 4, wobei die bekannte Konzentration des Nukleinsäure-Denaturierungsmittels im Bereich von etwa 5 Volumenprozent bis etwa 95 Vol.-% liegt.

6. Verfahren nach Anspruch 4, wobei das Nukleinsäure-Denaturierungsmittel Alkohol in einer Konzentration im Bereich von etwa 10 Vol.-% bis etwa 20 Vol.-% und das Nukleinsäure-Fällmittel (NH₄)₂SO₄ ist.

7. Verfahren nach einem der vorhergehenden Ansprüche, wobei der pH-Wert der wässrigen Reaktionslösung im Bereich von etwa 4 bis etwa 11 liegt.

8. Verfahren nach einem der vorhergehenden Ansprüche, wobei die Temperatur im Bereich von etwa 20°C bis etwa 90°C liegt.

9. Verfahren nach einem der Ansprüche 1 bis 5, 7 oder 8, wobei das Nukleinsäure-Fällmittel NaSO₄ ist.

10. Verfahren nach einem der Ansprüche 1 bis 5, 7 oder 8, wobei das Nukleinsäure-Fällmittel LiCl ist.

11. Verfahren nach einem der Ansprüche 1 bis 5, 7 oder 8, wobei das Nukleinsäure-Fällmittel (NH₄)₂SO₄ ist.

12. Verfahren nach einem der Ansprüche 1, 2, 4, 7 oder 8, wobei das Nukleinsäure-Fällmittel 13 Vol.-% bis 30 Vol.-% Sarkosyl ist.

13. Verfahren nach Anspruch 1, wobei eine wässrige Reaktionslösung, die das erste einzelsträngige Nukleinsäure-Molekül und das zweite einzelsträngige Nukleinsäure-Molekül enthält, mit einer Lösung gemischt wird, die das Nukleinsäure-Fällmittel enthält.

14. Analyseverfahren nach einem der Ansprüche 1 bis 13, wobei das zweite einzelsträngige Nukleinsäure-Molekül in einer klinischen Probe vorhanden ist und nachgewiesen wird, daß das zweite einzelsträngige Nukleinsäure-Molekül in der Probe vorhanden ist.

15. Verfahren zum Durchmustern eines vermuteten Nukleinsäure-Fällmittels zur Verwendung bei einer beschleunigten Nukleinsäure-Reassoziationsreaktion, wobei die Geschwindigkeit der Reassoziationsreaktion mindestens etwa das 100fache der Reaktionsgeschwindigkeit in einer Lösung unter Verwendung von 0,18 M NaCl bei 60°C ist, wobei das Durchmusterungsverfahren die folgenden Schritte umfaßt:
Herstellen einer Reihe von Lösungen, wobei jede der Lösungen eine bekannte Menge der einzelsträngigen Nukleinsäure und eine unterschiedliche Menge des vermuteten Nukleinsäure-Fällmittels enthält;
Untersuchen jeder der Lösungen auf das Vorhandensein eines Nukleinsäure-Präzipitats; und
Erhitzen jeder der Lösungen, bei denen das Nukleinsäure -Präzipitat gefunden wurde, um zu bestimmen, ob sich der Präzipitationsgrad verändert.

## Patentansprüche (Patentansprüche für folgende(n) Vertragsstaat(en): IT)

1. Verbessertes Verfahren zur Herstellung doppelsträngiger Nukleinsäure-Moleküle aus getrennten einzelsträngigen Nukleinsäure-Molekülen, wobei die Geschwindigkeit der Reaktion gegenüber der Reaktionsgeschwindigkeit unter Standardbezugsbedingungen stark erhöht ist, welches Verfahren die folgenden Schritte umfaßt:
Herstellen einer wässrigen Reaktionslösung, umfassend eine Quantität eines ersten einzelsträngigen Nukleinsäure-Moleküls,
- eine Quantität eines zweiten einzelsträngigen Nukleinsäure-Moleküls, welches zweite einzelsträngige Nukleinsäure-Molekül mindestens ein Segment der Basensequenzen aufweist, das komplementär zu einem entsprechenden Segment der Basensequenzen des ersten einzelsträngigen Nukleinsäure-Moleküls ist, wobei die Konzentration der zweiten Nukleinsäure eine derartige ist, daß zumindest eine 100fache Zunahme der Beschleunigung der Reaktionsrate bei Vorhandensein von 4 M LiCl, im Vergleich zur mit 0,18 M NaCl bei 60°C beobachteten Reaktionsgeschwindigkeit, beobachtet wird; und
- mindestens ein Nukleinsäure-Fällmittel, welches mit einer wässrigen Lösung mischbar und zum Ausfällen einzelsträngiger Nukleinsäuren aus einer wässrigen Lösung fähig ist, in einer ausreichenden Konzentration, einzelsträngigen Nukleinsäure-Moleküle mindestens etwa 100fach, im Vergleich zur Reaktionsgeschwindigkeit in Lösung unter Verwendung von 0,18 M NaCl bei 60°C, zu beschleunigen;
Inkubieren der wässrigen Reaktionslösung bei einer Temperatur, bei der die Reassoziation stattfinden kann; und
Analysieren der inkubierten wässrigen Reaktionslösung auf das Vorhandensein doppelsträngiger Nukleinsäure-Moleküle.

2. Verfahren nach Anspruch 1, wobei das Nukleinsäure-Fällmittel gewählt wird aus Reinigungsmittel, Dihydroxybenzol, Sarkosyl und den Alkalimetallsalzen und Ammoniumsalzen von SO₄, PO₄, C₁ und HCOO.

3. Verfahren nach Anspruch 1 oder 2, wobei das Nukleinsäure-Fällmittel in einer Konzentration im Bereich von etwa 1 M bis etwa 10 M bereitgestellt wird.

4. Verfahren nach einem der vorhergehenden Ansprüche, wobei die wässrige Reaktionslösung außerdem eine bekannte Konzentration eines Nukleinsäure-Denaturierungsmittels enthält.

5. Verfahren nach Anspruch 4, wobei die bekannte Konzentration des Nukleinsäure-Denaturierungsmittels im Bereich von etwa 5 Volumenprozent bis etwa 95 Vol.-% liegt.

6. Verfahren nach Anspruch 4, wobei das Nukleinsäure-Denaturierungsmittel Alkohol in einer Konzentration im Bereich von etwa 10 Vol.-% bis etwa 20 Vol.-% und das Nukleinsäure-Fällmittel (NH₄)₂SO₄ ist.

7. Verfahren nach einem der vorhergehenden Ansprüche, wobei der pH-Wert der wässrigen Reaktionslösung im Bereich von etwa 4 bis etwa 11 liegt.

8. Verfahren nach einem der vorhergehenden Ansprüche, wobei die Temperatur im Bereich von etwa 20°C bis etwa 90°C liegt.

9. Verfahren nach einem der Ansprüche 1 bis 5, 7 oder 8, wobei das Nukleinsäure-Fällmittel NaSO₄ ist.

10. Verfahren nach einem der Ansprüche 1 bis 5, 7 oder 8, wobei das Nukleinsäure-Fällmittel LiCl ist.

11. Verfahren nach einem der Ansprüche 1 bis 5, 7 oder 8, wobei das Nukleinsäure-Fällmittel (NH₄)₂SO₄ ist.

12. Verfahren nach einem der Ansprüche 1, 2, 4, 7 oder 8, wobei das Nukleinsäure-Fällmittel 13 Vol.-% bis 30 Vol.-% Sarkosyl ist.

13. Verfahren nach Anspruch 1, wobei eine wässrige Reaktionslösung, die das erste einzelsträngige Nukleinsäure-Molekül und das zweite einzelsträngige Nukleinsäure-Molekül enthält, mit einer Lösung gemischt wird, die das Nukleinsäure-Fällmittel enthält.

14. Analyseverfahren nach einem der Ansprüche 1 bis 13, wobei das zweite einzelsträngige Nukleinsäure-Molekül in einer klinischen Probe vorhanden ist und nachgewiesen wird, daß das zweite einzelsträngige Nukleinsäure-Molekül in der Probe vorhanden ist.

15. Verfahren zum Durchmustern eines vermuteten Nukleinsäure-Fällmittels zur Verwendung bei einer beschleunigten Nukleinsäure-Reassoziationsreaktion, wobei die Geschwindigkeit der Reassoziationsreaktion mindestens etwa das 100fache der Reaktionsgeschwindigkeit in einer Lösung unter Verwendung von 0,18 M NaCl bei 60°C ist, wobei das Durchmusterungsverfahren die folgenden Schritte umfaßt:
Herstellen einer Reihe von Lösungen, wobei jede der Lösungen eine bekannte Menge der einzelsträngigen Nukleinsäure und eine unterschiedliche Menge des vermuteten Nukleinsäure-Fällmittels enthält;
Untersuchen jeder der Lösungen auf das Vorhandensein eines Nukleinsäure-Präzipitats; und
Erhitzen jeder der Lösungen, bei denen das Nukleinsäure-Präzipitat gefunden wurde, um zu bestimmen, ob sich der Präzipitationsgrad verändert.
